## Europäisches Patentamt

## European Patent Office

## Office européen des brevets

(19)

(11) Veröffentlichungsnummer: **0 317 512 B1**

(12)

# EUROPÄISCHE PATENTSCHRIFT

(45) Veröffentlichungstag der Patentschrift:
**05.08.92 Patentblatt 92/32**

(51) Int. Cl.⁵: **A01G 7/00**, A01H 1/02,
C12N 5/00

(21) Anmeldenummer: **88810768.7**

(22) Anmeldetag: **09.11.88**

(54) **Eine leistungsfähige Methode, Baumwolle aus kultivierten Zellen zu regenerieren.**

(30) Priorität: **18.11.87 US 122162**

(43) Veröffentlichungstag der Anmeldung:
**24.05.89 Patentblatt 89/21**

(45) Bekanntmachung des Hinweises auf die
Patenterteilung:
**05.08.92 Patentblatt 92/32**

(84) Benannte Vertragsstaaten:
**AT BE CH DE ES FR GB GR IT LI LU NL SE**

(56) Entgegenhaltungen:
**EP-A- 262 971**
**WO-A-87/02701**
**US-A- 4 672 035**
**B.V.CONGER "Cloning Agri-cultural Plants Via In Vitro Techniques", 1981 CRC PRESS, Boca Raton Seite 196**

(56) Entgegenhaltungen:
**IN VITRO, Band 20, Nr. 3, Teil II, Juni 1984, Houston T.S.RANGAN "Somatic Embryo- - genesis in Tissue Cultures of Gossypiumhir-sutum" Seite 256**
**PLANT SCIENCE LETTERS, Band 32, Nr. 1,2, Oktober/November 1983, Amsterdam G.H.DA-VIDONIS "Plant Regeneration from CallusTis-sue of Gossypium Hirsutum" Seiten 89-93**
**IN VITRO, Band 13, Nr. 5, Mai 1977, Houston R.H.SMITH et al. "Defined Conditions for the Initiation and Growth of CottonCallus In Vitro" Seiten 329-334**

(73) Patentinhaber: **CIBA-GEIGY AG**
**Klybeckstrasse 141**
**CH-4002 Basel (CH)**

(72) Erfinder: **Finer, John**
**1515 Cleveland Road**
**Wooster Ohio 44691 (US)**

EP 0 317 512 B1

**Beschreibung**

Die vorliegende Erfindung ist auf Verfahren zur Regenerierung von Baumwollpflanzen aus kultivierten Zellen durch somatische Embryogenese gerichtet. Das Verfahren ist leistungsfähiger als die im Stand der Technik beschriebenen Verfahren.

Die somatische Embryogenese sollte von einem anderen Verfahren zur Pflanzenregenerierung, das als Organogenese bekannt ist, unterschieden werden. In beiden Techniken werden verpflanzte Gewebe von Quellen wie beispielsweise Wurzeln, Blätter oder Stengel kultiviert. Neue, differenzierte Gewebe bilden sich entweder direkt aus dem verpflanzten Gewebe oder aus undifferenziertem Kallusgewebe, das sich aus dem verpflanzten Gewebe entwickelt. In der Organogenese bestehen die neuen, differenzierten Gewebe aus Wurzeln und/oder Sprossen. In der somatischen Embryogenese andererseits sind die neuen differenzierten Gewebe bipolare Strukturen, die verbundene Wurzel- und Spross-meristematische Zentren, i.e. Embryonen, enthalten. Verschiedene Stadien können während der Embryonalentwicklung unterschieden werden. Diese Stadien umfassen solche, die dem Fachmann als globuläre, herz- und torpedoförmige sowie reife Stadien bekannt sind.

Unter passenden Bedingungen keimen die Embryonen und wachsen zu Pflänzchen heran, aus denen sich dann ausgewachsene Pflanzen bilden. Somatische Embryonen können in grossen Mengen produziert werden und sind deshalb für Massenvermehrung und Klonierung geeignet.

In einigen Fällen wurde gefunden, dass sich die Zellen von ausgewachsenen Pflanzen, die durch somatische Embryogenese hergestellt wurden, genetisch von den Zellen unterscheiden, die benutzt wurden, um den embryogenen Kallus herzustellen. Von einer Pflanzenzelle, die auf diese Weise verändert ist, wird gesagt, dass sie eine genetische Veränderung, die als somaklonale Variation bekannt ist, durchgemacht hat. In einigen Fällen sorgt der neue Genotyp für eine erwünschte Eigenschaft. Die Erzeugung modifizierter Genotypen mit Hilfe von Pflanzenzellkulturtechniken stellt eine weitere Verwendung der somatischen Embryogenese dar.

Somatische Embryogenese könnte auch benutzt werden, um Pflanzen aus kultivierten Zellen zu regenerieren, die wegen einer bestimmten Eigenschaft ausgewählt wurden. Beispielsweise könnten kultivierte Zellen einem Phytotoxin ausgesetzt werden, gegen das eine Resistenz gewünscht wird. Die somatische Embryogenese könnte dann benutzt werden, um Pflanzen aus den Zellen zu regenerieren, die das Phytotoxin am besten tolerieren. Solche in vitro Selektion, die kultivierte Zellen und Gewebe auf diese Weise nutzt, wurde von Chaleff und Ray in Science 223, 1148-1151 (1984) beschrieben.

Es hat auch Berichte gegeben, Baumwollpflanzen durch somatische Embryogenese aus Gewebe zu regenerieren. Beispielsweise berichten Rangan et al. [In Vitro 20, 256 (1984)] über die erfolgreiche Regenerierung von Baumwolle (*Gossypium hirsutum*) durch somatische Embryogenese. Davidonis und Hamilton [Plant Science Letters 32, 89 (1983)] stellten ein paar Pflanzen aus somatischen Proembryoiden von G. *hirsutum* her, die sie aus zwei Jahre altem Kallusgewebe erhalten hatten. Finer et al. [TCA Report 17, 8 (1983)] regenerierten eine Pflanze aus somatischen Embryonen von G. *klotzschianum*. Smith et al. [In Vitro 13, 329 (1977)] regenerierten ein Pflänzchen aus dem Kallus eines Keimblatts von G. *arboreum*. Stuart und Strickland (WO 87/02701) beschreiben den Einfluss von Variationen der Stickstoffquellen in den Kulturmedien bei der somatischen Embryogenese auf die Quantität und Qualität von dabei erhaltenen Embryonen, u. a. Baumwoll-Embryonen. Dieses Dokument enthält alle Merkmale des Oberbegriffes des Patentanspruches 1.

Die im Stand der Technik beschriebenen Verfahren sind insofern unzulänglich, als sie nicht Pflanzen in der grossen Anzahl produzieren, die für kommerzielle Anwendungen benötigt werden. Die Fähigkeit, Pflanzen aus kultivierten Zellen zu regenerieren, hat nur dann einen praktischen Gebrauchswert, wenn die Bildung von Vermehrungseinheiten und die Regenerierung von Pflanzen aus Vermehrungseinheiten leistungsstark ist. Deshalb werden leistungsfähigere Verfahren zur Regenerierung von Baumwollpflanzen durch somatische Embryogenese benötigt.

Gegenstand der Erfindung

Gegenstand der vorliegenden Erfindung sind Verfahren, leistungsfähiger als die des Standes der Technik, um somatische Embryonen aus kultivierten Baumwollzellen herzustellen und um Pflanzen aus solchen Embryonen zu regenerieren. Ein weiterer Gegenstand der vorliegenden Erfindung sind Verfahren der somatischen Embryogenese, wobei ein Zellsuspensionskultursystem benutzt wird.

Zusammenfassung der Erfindung

Diese und andere Gegenstände der vorliegenden Erfindung wurden durch die Vorlage eines Verfahrens zur Herstellung proembryonaler Baumwollzellmassen in flüssigen Suspensionen ermöglicht, das folgende Schritte aufweist:

(a) Induktion der Baumwollkallusbildung bei etwa 20° bis 40°C, indem Baumwollpflanzengewebe auf ein Medium, das zur Kallusinduktion geeignet ist, aufgelegt wird, mit nachfolgenden weiteren Kultivierungsschritten zur Verhinderung von Bräunung;

(b) Suspension von bis zu 40 mg/ml des Kallus in einem geeigneten flüssigen Medium, das proembryonale Zellmassen induziert, welches eine relativ niedrige Konzentration von wenigstens einem Auxin enthält, bis sich klumpige Zusammenballungen proembryonaler Zellmassen bilden, die sich schnell vermehren; und dadurch gekennzeichnet, daß der zusätzliche Schritt durchgeführt wird:

(c) Uebertragung der sich rasch vermehrenden klumpigen Zusammenballungen der proembryonalen Zellmassen in ein flüssiges Nährmedium, in welchem kleinere, feiner dispergierte proembryonale Zellmassen gebildet werden können, wobei das besagte Nährmedium wenigstens ein Auxin enthält, in einer Konzentration die höher ist als die, die gewöhnlicherweise in Suspensionskulturmedien verwendet wird und die auf jeden Fall bedeutend höher ist als die entsprechende Auseinkonzentration in Schritt b).

Die proembryonalen Zellmassen sind befähigt, sich in reife Embryonen, kleine Pflänzchen und letztlich ausgewachsene Pflanzen zu entwickeln.

## Abbildungen

Die Abbildungen 1 bis 8 zeigen die verschiedenen Stadien der vorliegenden Erfindung.

Fig. 1 ist die Photographie eines Kallus, der durch das weiter unten in Schritt (a) beschriebene Verfahren hergestellt wird, indem als Quelle somatische Embryonen verwendet werden. Die Photographie ist 6,2 mal vergrössert. Der Balken von 1,2 cm stellt 2 mm dar.

Fig. 2 ist die Photographie eines Kallus, der durch das weiter unten in Schritt (a) beschriebene Verfahren hergestellt wird, indem als Quelle Keimblätter verwendet werden. Die Photographie ist 22,7 mal vergrössert. Der Balken von 1,2 cm stellt 0,5 mm dar.

Fig. 3 ist die Photographie klumpiger Zusammenballungen von proembryonalen Zellmassen, die durch das in Schritt (b) beschriebene Verfahren hergestellt werden. Die Photographie ist 44,4 mal vergrössert. Der Balken von 1,1 cm stellt 0,25 mm dar.

Fig. 4 ist die Photographie fein dispergierter proembryonaler Zellmassen, die durch das in Schritt (c) beschriebene Verfahren hergestellt werden. Die Photographie ist 44,4 mal vergrössert. Der Balken von 1,1 cm stellt 0,25 mm dar.

Fig. 5 bis 8 sind jeweils Photographien von globulären, herz- und torpedoförmigen sowie reifen Embryonen, die durch das in Schritt (d) dieser Erfindung beschriebene Verfahren hergestellt werden. Die Photographien sind 2,7 mal vergrössert. Der Balken von 1,8 cm in jeder Photographie stellt 0,5 mm dar.

## Detaillierte Beschreibung der Erfindung

Es wurde überraschend gefunden, dass sich zur Keimung und zur Regenerierung von Pflanzen befähigte Baumwoll (Gossypium spp.)-Embryonen in grosser Zahl durch somatische Embryogenese herstellen lassen, dadurch, dass man von proembryonalen Zellmassen ausgeht und daraus in einem Zellsuspensionskultursystem Embryonen heranzieht.

Mit dem vorliegenden Verfahren kann man beispielsweise in einem Standard-250 ml-Delong-Gefäss etwa 10 000 globuläre Embryonen erzeugen, aus denen etwa 1000 reife Embryonen und daraus wiederum etwa 50 Pflanzen entstehen. Die Leistungsfähigkeit der Verfahren des Standes der Technik ist deutlich geringer.

Die Baumwollpflanzen, die gemäss vorliegender Erfindung hergestellt werden, können Zuchtformen oder Wildformen sein. Zuchtformen sind bevorzugt.

Einige Beispiele von Baumwollzuchtformen umfassen G. hirsutum, G. arboreum und G. barbadense. G. hirsutum ist bevorzugt. Einige Sorten von G. hirsutum, die gemäss dem Verfahren vorliegender Erfindung regeneriert werden können, umfassem Coker 310, Coker 312, Acala SJ2, Acala SJ4, Acala SJ5, Funk 519-2 und Funk 522-1. Die bevorzugte Sorte ist Coker 310.

## Schritt a: Embryogener Baumwollkallus

Der erste Schritt im vorliegenden Verfahren besteht in der Induktion der Baumwollkallusbildung aus verpflanztem Baumwollgewebe. Einige Beispiele von geeignetem verpflanztem Baumwollgewebe sind somatische Embryonen, reife und unreife zygotische Embryonen, Keimblätter oder Hypokotyle eines Sämlings und junges Gewebe einer reifen Pflanze. Somatische Embryonen und Sämlingskeimblätter oder -hypokotyle sind dabei bevorzugt.

EP 0 317 512 B1

Beispielsweise können zygotische Embryonen durch Herausschneiden aus Samenanlagen erhalten werden. Die Samenanlagen werden vorzugsweise etwa 7 bis 30 Tage nach der Bestäubung herausgeschnitten, vorzugsweise etwa 10 bis 21 Tage nach der Bestäubung und insbesondere etwa 12 bis 16 Tage nach der Bestäubung.

Keimblätter und Hypokotyle kann man jungen Sämlingen entnehmen. Die Sämlinge sind normalerweise etwa 3 bis 21 Tage alt, bevorzugt etwa 4 bis 9 Tage, insbesondere etwa 7 Tage alt. Hypokotyle werden längs aufgeschnitten und in passende Abschnitte geteilt, beispielsweise in Abschnitten von 1 bis 20 mm, bevorzugt von etwa 2 mm Länge. Das Keimblattgewebe wird in Stücke mit einer Fläche von etwa 1 bis 400 mm$^2$ geschnitten, bevorzugt von etwa 5 bis 100 mm$^2$, insbesondere von etwa 10 mm$^2$.

Somatische Embryonen, die sich von dieser Vorgehensweise ableiten, sind eine ganz besonders bevorzugte Quelle, um embryogenen Kallus gemäss dem vorliegenden Verfahren zu erhalten.

Somatische Embryonen können beispielsweise erhalten werden, indem das Verfahren angewendet wird, das oben für Hypokotyl- und Keimblattgewebe als Quelle des verpflanzten Gewebes beschrieben ist. Jeder somatische Embryo, der vor der Entfaltung des Primärblattes genommen wird, ist geeignet. Die Grösse des somatischen Embryos ist nicht kritisch. Bevorzugt sind jedoch somatische Embryonen von weniger als 5 mm Länge.

Junggewebe von ausgewachsenen Baumwollpflanzen wird normalerweise dadurch erhalten, dass man die apikalen 10 cm, vorzugsweise etwa 5 cm der Sprossspitze entnimmt. Stengel und Petiolengewebe werden längs aufgeschnitten und in Abschnitte derselben Grösse wie bei den Hypokotylen (siehe oben) geteilt. Blattgewebe wird in gleich grosse Stücke geschnitten wie das Keimblattgewebe (siehe oben).

Das Baumwollpflanzengewebe wird auf ein zur Kallusinduktion geeignetes Medium, bei etwa 20° bis 40°C, vorzugsweise 23° bis 35°C, insbesondere etwa 31°C gelegt. Jedes Medium, das aus einem Gewebe einen Kallus induzieren kann, kann im vorliegenden Verfahren eingesetzt werden. Das Medium kann flüssig oder fest sein, obwohl ein festes Medium bevorzugt ist, weil es bequemer zu handhaben ist.

Ein gebräuchliches, die Kallusbildung induzierendes Medium enthält im allgemeinen anorganische Salze sowie Vitamine, eine Kohlenstoffquelle, ein Auxin und ein Cytokinin. Dieses Medium wird auf einen pH zwischen 3,5 und 7,5, bevorzugt zwischen 4,5 und 6,5, insbesondere auf etwa 5,7 eingestellt.

Es sind an sich alle anorganischen Salze sowie Vitamine geeignet, die die Kallusinduktion fördern. Einige Beispiele von geeigneten anorganischen Salzen und von Vitaminen werden von Murashige und Skoog in Physiol. Plant 15, 473-497 (1962) (MS) sowie Gamborg et al. in Exp. Cell Res. 50, 151-158 (1968) (B-5) beschrieben. Ein weiteres Beispiel dafür ist die Modifikation des MS- oder Gamborgs B-5-Mediums, das Cheng et al., Plant Sci. Lett. 19, 91-99 (1980) beschreiben. Die bevorzugten anorganischen Salze sind MS anorganische Salze. Die bevorzugten Vitamine sind Gamborgs B-5 Vitamine.

Als geeignete Kohlenstoffquelle kann jede Kohlenstoffquelle eingesetzt werden, auf der Kallus wachsen kann. Bevorzugte Kohlenstoffquellen sind Zucker und Derivate von Zuckern. Bevorzugte Zucker sind Glucose und Saccharose. Es ist besonders wünschenswert, Kallus in einem Kallusinduktionsmedium zu initiieren, das Glucose enthält, damit das Braunwerden des Gewebes verringert wird, und dann den Kallus in ein Kallusinduktionsmedium, das Saccharose enthält, zu übertragen.

Die Konzentration der Kohlenstoffquelle ist 5 bis 60 g/Liter, bevorzugt etwa 30 g/Liter.

Das im Kallusinduktionsmedium vorhandene Auxin kann jedes Auxin sein, das einen Kallus induzieren kann. Einige geeignete Auxine umfassem α-Naphthalinessigsäure, Picloram, 2,4,5-Trichlorphenoxyessigsäure, 2,4-Dichlor-phenoxyessigsäure, Indol-3-buttersäure, Indol-3-milchsäure, Indol-3-bernsteinsäure, Indol-3-essigsäure und p-Chlorphenoxyessigsäure, Indol-3-essigsäure und p-Chlorphenoxyessigsäure. Ein bevorzugtes Auxin ist α-Naphthalinessigsäure.

Jede Auxinkonzentration, die die Kallusbildung induzieren kann, kann im vorliegenden Verfahren verwendet werden. Besonders geeignete Konzentrationen liegen bei 0,1 bis 10 mg/Liter, insbesonders wenn α-Naphthalinessigsäure als Auxin eingesetzt wird.

Das im Kallusinduktionsmedium vorhandene Cytokinin kann jedes Cytokinin sein, das einen Kallus induzieren kann. Einige geeignete Cytokinine umfassen Kinetin, 6-Benzyladenin, 2-Isopentenyladenin und Zeatin. Ein bevorzugtes Cytokinin ist Kinetin.

Jede Cytokininkonzentration, die die Kallusbildung induzieren kann, kann im vorliegenden Verfahren eingesetzt werden. Geeignete Konzentrationen liegen bei 0,1 bis 10 mg/Liter. Eine bevorzugte Konzentration ist 1 mg/Liter, insbesonders wenn das Cytokinin Kinetin ist.

Wenn das Medium fest ist, enthält es als Verfestigungsmittel beispielsweise etwa 0,8 % Agar, wie Agar Noble (Difco), oder etwa 0,8 % Agarose. (Alle Prozentangaben in dieser Beschreibung beziehen sich auf das Gewicht.)

Das Gewebe wird einige Zeit auf dem Kallusinduktionsmedium kultiviert, und zwar so lange, bis sich der Kallus bildet. Man kann beispielsweise Gewebe auf einem Kallusinduktionsmedium kultivieren, das Glucose

4

als Kohlenstoffquelle enthält. Eine fünfwöchige Induktionsdauer ist typisch. Überimpfungen in frisches Medium und weitere Kultivierung werden durchgeführt, weil sie nötig sind, um Braunwerden zu verhindern. Wöchentliche Überimpfungen sind bevorzugt.

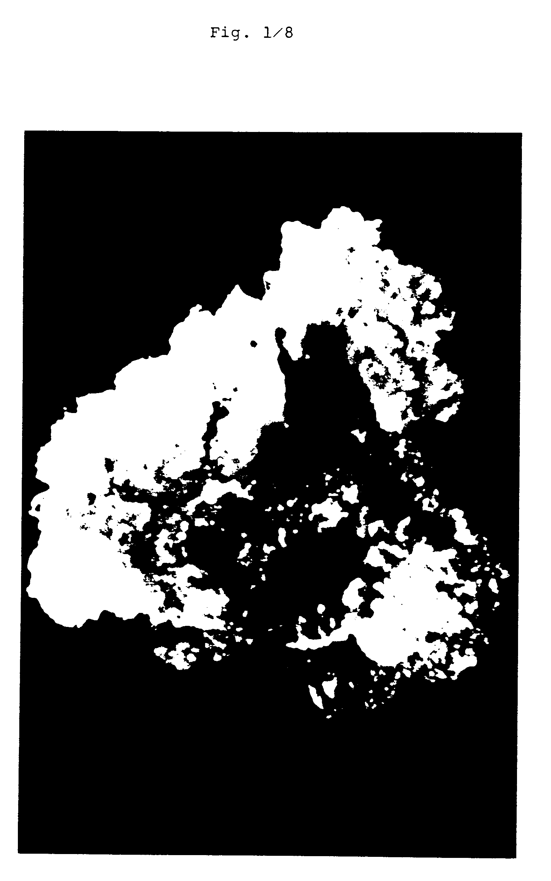

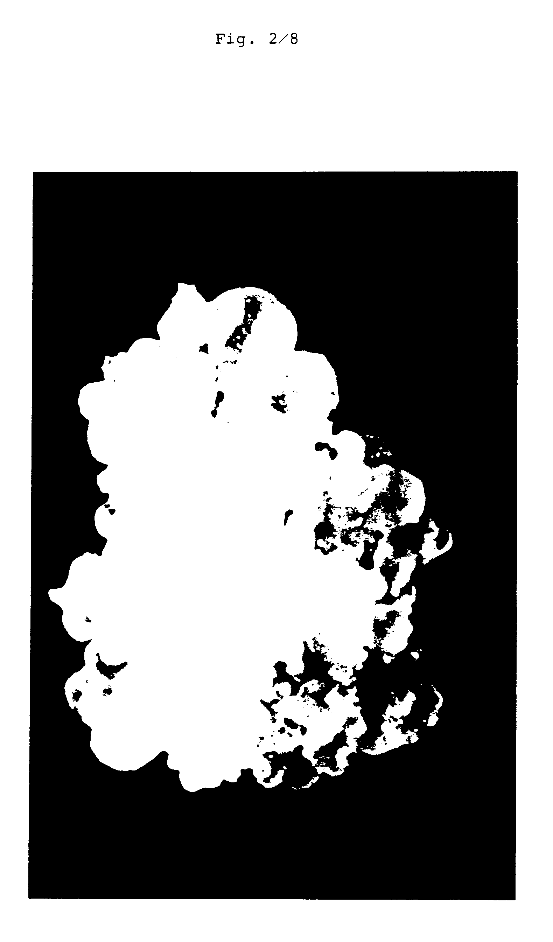

Der Kallus, der sich bildet, kann unorganisiert sein, oder er kann proembryonale Zellmassen, embryogenen Kallus oder auch Embryonen enthalten. Normalerweise, wenn Hypokotyle oder Keimblätter als Quelle für das verpflanzte Gewebe verwendet werden, scheint sich bevorzugt unorganisierter Kallus zu bilden. Wenn somatische Embryonen als Quelle für das verpflanzte Gewebe verwendet werden, scheint wenigstens ein Teil des Kallus aus einem embryogenen Kallus zu bestehen, welcher durch eine leicht gelbe Farbe und Knotenbildung charakterisiert ist. Photographien von Kalli, die durch Schritt (a) aus somatischen Embryonen und aus Keimblättern hergestellt wurden, werden in Fig. 1 bzw. Fig. 2 wiedergegeben.

Der resultierende Kallus kann anschliessend für eine Zeitspanne von bis zu 5 Monaten vorteilhafterweise auf ein Kallusweiterkulturmedium übertragen werden, das dem Kallusinduktionsmedium ähnlich ist, aber Saccharose als Kohlenstoffquelle enthält. Man kultiviert den Kallus vorzugsweise für einen Zeitraum von einem Monat auf einem Kallusinduktionsmedium, das Saccharose enthält, oder für einen Zeitraum von zwei Monaten, sollte dann jedoch nach einem Monat auf frisches Medium überimpfen.

Der Kallus kann im Dunkeln induziert werden, wird aber vorzugswiese im Licht induziert. Das Licht kann eine Intensität von beispielsweise 0,5 bis 150 $\mu$E m$^{-2}$s$^{-1}$ (= 41,75 bis 12525 1x) haben.

Schritt (b): Klumpige Zusammenballungen von proembryonalen Zellmassen

Der Kallus von Schritt (a) wird in einem Flüssigmedium suspendiert, das die Entwicklung von proembryonalen oder sich vermehrenden embryonalen Zellmassen fördert. Es ist wichtig, dass die Zelldichte niedrig ist. Deshalb wird nicht mehr als 40 mg Kallus/ml Kulturmedium, bevorzugt nicht mehr als 15 mg Kallus/ml Kulturmedium und insbesondere nicht mehr als 5 mg Kallus/ml Kulturmedium suspendiert.

Das Medium, brauchbar in Schritt (b), kann jedes Medium sein, das proembryonale Zellmassen induzieren kann. Das Medium enthält im allgemeinen anorganische Salze sowie Vitamine, eine Kohlenstoffquelle und ein Auxin. Das Medium kann auch organische Stickstoffquellen, Cytokinine, Aminosäuren und andere Zusätze enthalten, wie Kaseinhydrolysat oder Kokosmilch.

Die anorganischen Salze und die Vitamine können die gleichen sein wie unter Schritt (a), weiter oben. MS anorganische Salze und B-5-Vitamine sind bevorzugt.

Die Kohlenstoffquelle kann die gleiche sein wie die in Schritt a beschriebene. Saccharose ist bevorzugt. Die Konzentration der Kohlenstoffquelle ist 0,1 bis 100 g/Liter. Etwa 20 g/Liter ist bevorzugt, besonders, wenn die Kohlenstoffquelle Saccharose ist.

Das Auxin kann aus den Auxinen, die in Schritt (a) verwendet werden, ausgewählt sein. Die bevorzugten Auxine sind 2,4-Dichlorphenoxyessigsäure und Picloram. Picloram ist besonders bevorzugt.

Die Konzentration des Auxins in Schritt (b) ist relativ niedrig. Die genaue Konzentration hängt von dem spezifischen, verwendeten Auxin ab. Die relativ niedrige Auxinkonzentration ist im allgemeinen ähnlich der, die gewöhnlicherweise in Suspensionskulturmedien verwendet wird, und ist bedeutend niedriger als die entsprechende Auxinkonzentration, die in Schritt (c) verwendet wird. Wenn Picloram das in Schritt (b) verwendete Auxin ist, ist die Konzentration 0,01 bis 5 mg/Liter, bevorzugt 0,1 bis 1 mg/Liter und insbesondere etwa 0,5 mg/Liter. Wenn 2,4-Dichlorphenoxyessigsäure das in Schritt (b) verwendete Auxin ist, ist die Konzentration 0,01 bis 0,5 mg/Liter, bevorzugt 0,05 bis 0,25 mg/Liter und insbesondere etwa 0,1 mg/Liter.

Die Induktion proembryonaler Zellmassen wird vozugsweise in einem belüfteten Medium bei einer Temperatur zwischen 20° und 35°C durchgeführt. Bevorzugt sind Temperaturen zwischen 22° und 33°C und insbesondere zwischen 25° und 31°C. Das Medium kann auf jede im Stand der Technik bekannte Weise belüftet werden, beispielsweise durch Schütteln. Schritt (b) kann im Dunkeln durchgeführt werden oder im Licht von bis zu 75 $\mu$E m$^{-2}$s$^{-1}$ (= 6262,5 1x), bevorzugt zwischen 5 und 10 $\mu$E m$^{-2}$s$^{-1}$ (= 417,5 und 835 1x).

Der Kallus wird vorzugsweise ohne Ueberimpfen in dem Medium gehalten, bis sich klumpige Zusammenballungen von proembryonalen Zellmassen bilden und beginnen, sich rasch zu teilen. Der Ausbruch rascher Teilung erfolgt gewöhnlich nach 3 bis 8 Wochen, typischerweise nach 5 bis 7 Wochen. Während der Induktionsperiode kann das Medium an sich durch frisches Medium ersetzt werden, obwohl es vorzuziehen ist, das Medium während dieser Periode nicht zu ersetzen.

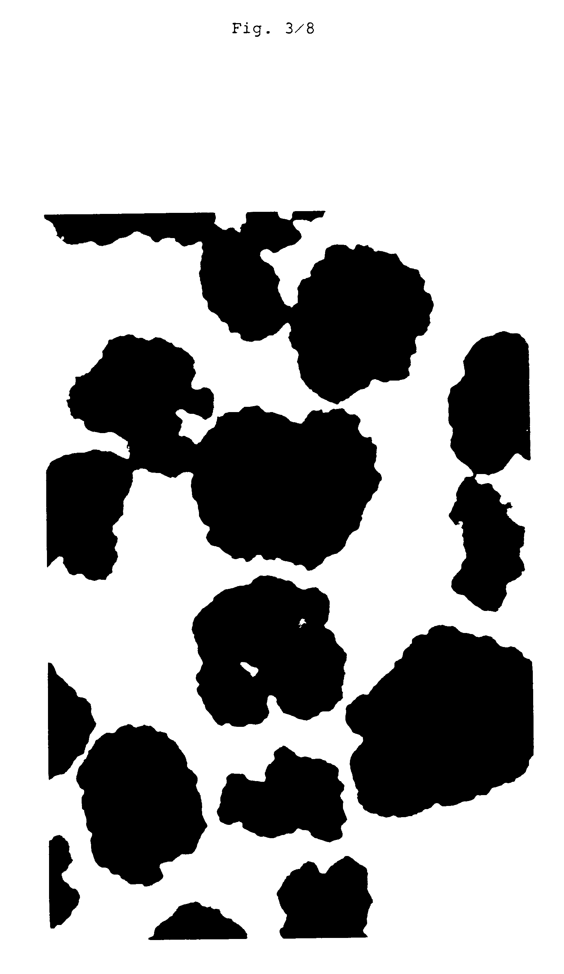

Der Wechsel von Kallus zu klumpigen Zusammenballungen proembryonaler Zellmassen ist für den Fachmann der Pflanzengewebekultur leicht erkennbar. Er ist durch die leicht gelbe Farbe und die klumpige Natur der proembryonalen Zellmassen gekennzeichnet. Eine Photographie solcher Zellmassen wird in Fig. 3 wiedergegeben.

Wenn die klumpigen Zusammenballungen der proembryonalen Zellmassen erst einmal begonnen haben, sich rasch zu teilen, können sie direkt in das in Schritt (c) beschriebene Medium eingebracht werden oder in frisches Medium überimpft werden, um das Braunwerden zu verhindern. Ein Ueberimpfen im Zeitraum von 3 bis 7 Tagen, bevorzugt alle 5 bis 7 Tage ist günstig. Ohne Ueberimpfen überleben die Zellmassen etwa vierzehn Tage.

Schritt c: Fein dispergierte proembryonale Zellmassen

Die klumpigen Zusammenballungen proembryonaler Zellmassen von Schritt (b) werden in ein Flüssigmedium übertragen, das dazu beiträgt, dass die klumpigen Zusammenballungen der proembryonalen Zellmassen fein dispergiert sind. Das Medium kann dem in Schritt (b) beschriebenen ähnlich sein, sollte aber eine relativ hohe Auxinkonzentration aufweisen. Als Auxin kann jedes in Schritt (a) verwendete Auxin eingesetzt werden. Bevorzugte Auxine sind 2,4,5-Trichlorphenoxyessigsäure und 2,4-Dichlorphenoxyessigsäure. Insbesonders bevorzugt ist 2,4-Dichlorphenoxyessigsäure.

Die Auxinkonzentration hängt von dem einzelnen Auxin ab. Die Auxinkonzentration im Medium von Schritt (c) ist generell höher oder wenigstens am oberen Ende des Konzentrationsbereichs, der gewöhnlicherweise in Suspensionskulturmedien verwendet wird; und ist auf jeden Fall bedeutend höher als die entsprechende Auxinkonzentration in Schritt (b).

Wenn beispielsweise 2,4-Dichlorphenoxyessigsäure als Auxin in Schritt (c) eingesetzt wird, kann die Konzentration bei etwa 0,5 bis 100 mg/Liter liegen, bevorzugt bei 1 bis 10 mg/Liter und insbesondere bei etwa 2,5 bis 7,5 mg/Liter.

Bis auf die Konzentration und möglicherweise das gewählte Auxin können Medium, Temperatur und Lichtmenge in Schritt (c) gleich sein wie in Schritt (b).

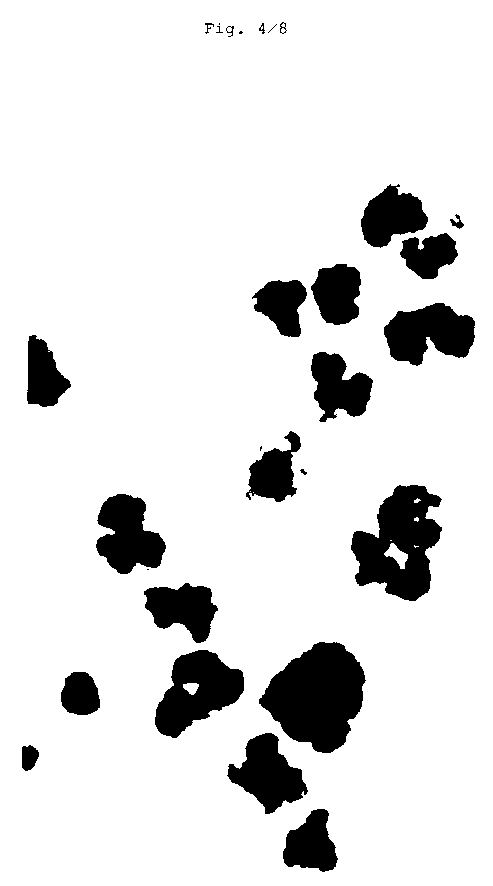

Die Bedingungen von Schritt (c) werden aufrecht erhalten, bis die klumpigen Zusammenballungen der proembryonalen Zellmassen zu kleineren, feiner dispergierten proembryonalen Zellmassen werden. Das Erscheinen der kleineren, feiner dispergierten proembryonalen Zellmassen ist für den Fachmann leicht erkennbar. Diese Zellmassen sind durch ihre gelbe Farbe, glatte Oberfläche, mittlere Dichte und geringe Grösse charakterisiert. Eine Photographie der Zellmassen wird in Fig. 4 wiedergegeben. Der Uebergang zu den kleineren, feiner dispergierten Zellmassen geschieht gewöhnlich innerhalb von 6 Wochen, typischer noch innerhalb von 2 Wochen.

Die Kultur der kleinen, fein dispergierten proembryonalen Zellmassen kann unbegrenzt aufrecht erhalten werden, und kann überimpft werden, um aktives Wachstum zu erhalten. Es ist günstig, beispielsweise alle 3 bis 28 Tage, bevorzugt alle 5 bis 10 Tage zu überimpfen.

Schritt d: Reife Embryonen

Die kleineren, feiner dispergierten proembryonalen Zellmassen werden zu einem Medium, das die Entwicklung reifer Embryonen induziert, hinzugefügt. Das Medium ist vorzugsweise flüssig.

Embryonen durchlaufen eine Anzahl von Entwicklungsstadien, bevor sie reif und fähig sind, zu keimen. Diese Stadien umfassen globuläre, herz- und torpedoförmige sowie reife Stadien. Die Bezeichnungen der Stadien basieren auf dem ungefähren Aussehen der Embryonen.

Das in Schritt (d) brauchbare Medium kann jedes Medium sein, dass die Entwicklung reifer Embryonen zu induzieren vermag. Ein brauchbares Medium enthält im allgemeinen anorganische Salze sowie Vitamine, eine Kohlenstoffquelle und eine organische Verbindung, die reduzierten Stickstoff enthält.

Die Salze und Vitamine sowie deren Konzentrationen können die gleichen sein wie bei Schritt (a). Die Kohlenstoffquelle kann auch wie in Schritt (a) gewählt sein. Die Konzentration der Kohlenstoffquelle ist etwa 1 bis 10 g/Liter, bevorzugt etwa 2 bis 6 g/Liter. Eine bevorzugte Kohlenstoffquelle ist Saccharose.

Die organische Verbindung, die reduzierten Stickstoff enthält, kann jede solche Verbindung sein, die, wenn sie dem Medium von Schritt d hinzugefügt wurde, die Entwicklung reifer Embryonen induziert. Die bevorzugten Verbindungen sind Aminosäuren. Eine bevorzugte Aminosäure ist Glutamin.

Die Konzentration der organischen Quelle von freiem Stickstoff hängt von der eingesetzten Verbindung ab. Eine wirksame Konzentration von Glutamin als organischer Quelle von reduziertem Stickstoff liegt bei 2 bis 260 mM, bevorzugt 5 bis 100 mM, insbesondere 10 bis 50 mM.

Das Medium von Schritt (d) kann ein Auxin enthalten. Auxine sind erstrebenswert während der frühen Stadien der Embryonalentwicklung, aber nicht während der späteren Stadien. Deshalb sind sie, falls überhaupt anwesend, vorzugsweise nur bis zum herzförmigen Stadium der Entwicklung einzusetzen. Danach werden die Embryonen in ein Medium, das kein Auxin enthält, übertragen.

Falls vorhanden, kann die Auxinkonzentration bei 0,01 bis 0,1 mg/Liter liegen.

Das Auxin kann eines der in Schritt (a) brauchbaren Auxine sein. Die bevorzugten Auxine sind Picloram und 2,4-Dichlorphenoxyessigsäure.

Die Embryonen können sich im Medium von Schritt (d) bei Temperaturen von 20° bis 35°C im Dunkeln oder im Licht entwickeln. Die Lichtintensität kann beispielsweise 5 bis 75 $\mu$E m$^{-2}$s$^{-1}$ (= 6262,5 1x) betragen.

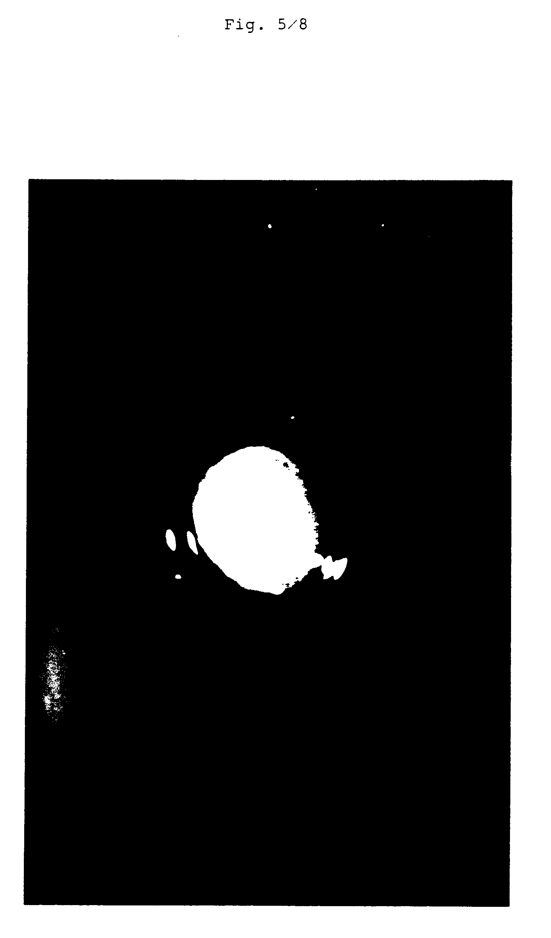

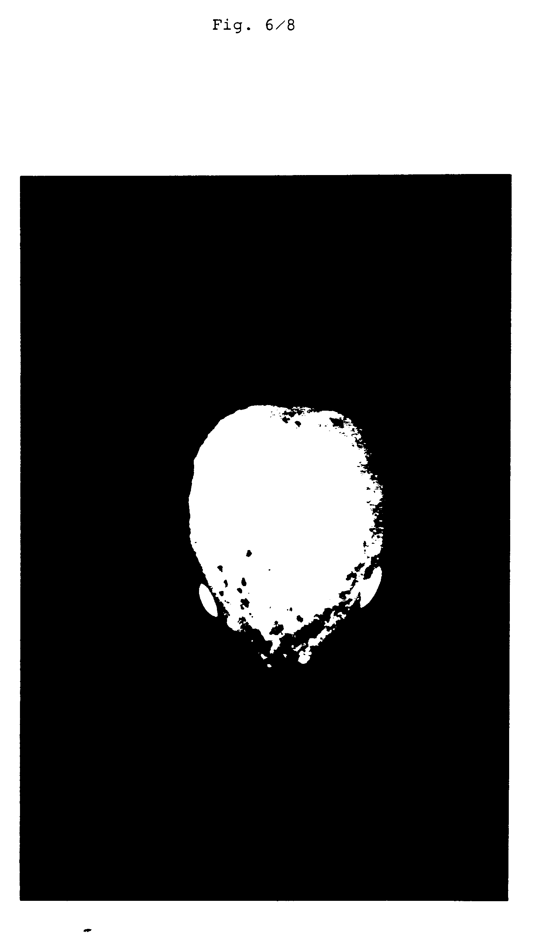

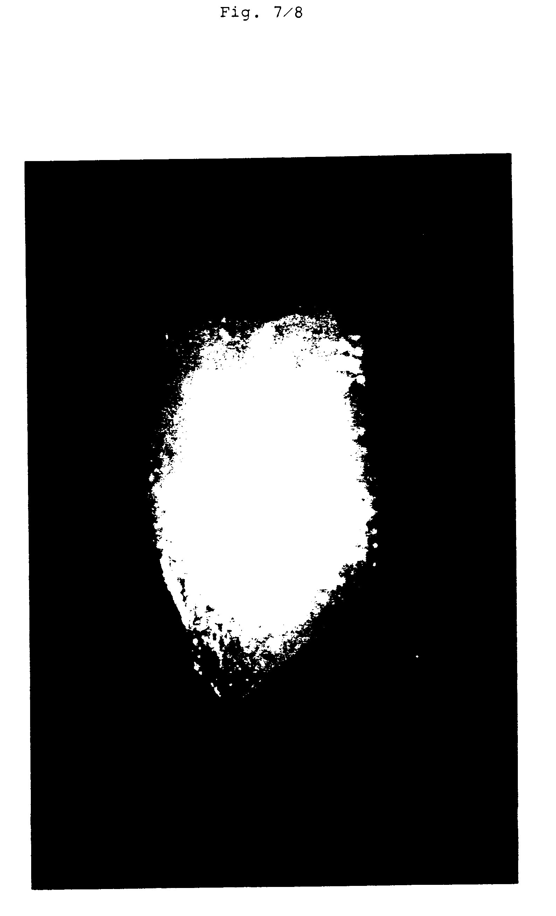

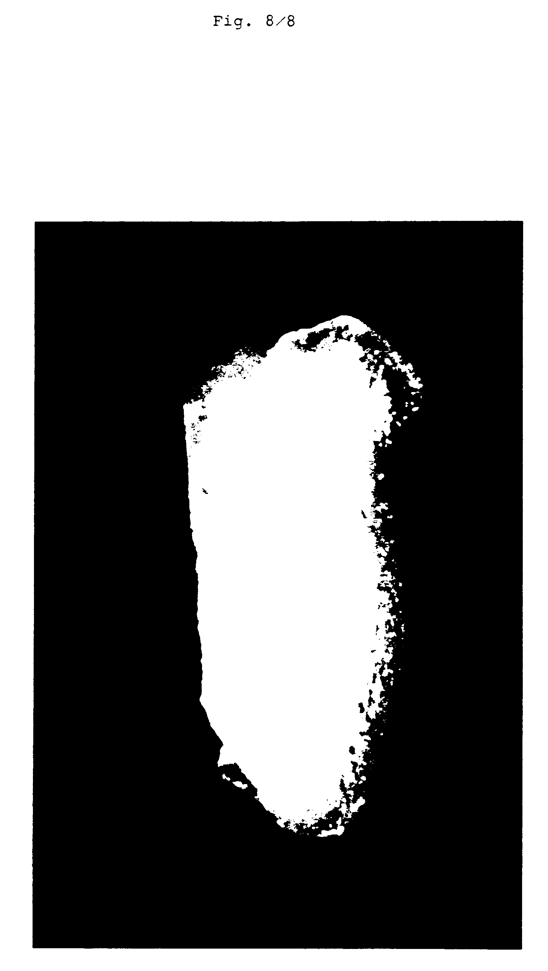

Die Embryonen werden im Medium von Schritt (d) gehalten, bis die Embryonen zu torpedoförmigen oder reifen Stadien herangewachsen sind. Für den mit Pflanzengewebekulturen umgehenden Fachmann sind die globulären, herzund torpedoförmigen sowie reifen Embryonen klar und einfach zu erkennen. Photographien dieser Embryonen sind in Fig. 5 bis 8 wiedergegeben. Die Embryonen reifen typischerweise in etwa 2 bis 5 Wochen, gewöhnlicherweise in etwa 3 bis 4 Wochen. Es ist gewöhnlich unnötig, die Embryonen ein weiteres Mal zu kultivieren bzw. die Embryonen in frisches Medium zu übertragen. Nur im herzförmigen Stadium könnte es vorteilhaft sein, wenn man von einem Auxin-enthaltenden Medium zu einem Medium, das kein Auxin enthält, überwechselt.

### Schritt e: Keimung

Die reifen Embryonen werden auf ein festes Medium aufgebracht, das Keimung induziert. Das Medium enthält anorganische Salze sowie Vitamine und eine Kohlenstoffquelle. Das Medium ist mit einem gebräuchlichen Verfestigungsmittel, wie Gelrite (Kelko, San Diego, Kalifornien), Agarose oder Agar, verfestigt.

Die anorganischen Salze können diejenigen aus Schritt (a) sein, wobei das Nitrat in hohen Konzentrationen vorliegt, während Ammonium fehlt oder in sehr niedrigen Konzentrationen vorliegen kann. Die Nitratkonzentration beträgt normalerweise 20 bis 60 mM, bevorzugt 30 bis 60 mM, insbesondere 35 bis 45 mM. Die Konzentration der Ammoniumionen sollte 5 mM nicht übersteigen.

Als Kohlenstoffquelle kommt vorzugsweise ein Zucker in Frage. Ein bevorzugter Zucker ist Saccharose. Die Konzentration der Kohlenstoffquelle hängt von der Art der verwendeten Kohlenstoffquelle ab. Wenn beispielsweise Saccharose als Kohlenstoffquelle eingesetzt wird, beträgt deren Konzentration 0,1 bis 6 % (Gewichtsprozente), bevorzugt 0,5 bis 4 %, insbesondere 1 bis 3 %.

Im Medium von Schritt (e) ist fakultativ eine organische Verbindung, die reduzierten Stickstoff enthält, vorhanden. Als bevorzugte organische Verbindungen kommen Aminosäuren oder deren Gemische in Frage, die befähigt sind, die Keimung zu unterstützen. Zu den bevorzugten Aminosäuren oder Mischungen davon gehören Glutamin und Kaseinhydrolysat.

Die Konzentration der organischen Quelle des reduzierten Stickstoffs hängt im allgemeinen von der Art der speziell verwendeten Verbindung ab. Wenn die Verbindung beispielsweise Glutamin ist, kann deren Konzentration 2 bis 50 mM sein, liegt jedoch bevorzugt bei 5 bis 30 mM, insbesondere bei 10 bis 20 mM. Wenn die Verbindung Kaseinhydrolysat oder modifiziertes Kaseinhydrolysat ist, liegt die Konzentration bei 100 bis 3000 mg/Liter, bevorzugt 1000 bis 2800 mg/Liter, insbesondere bei 1500 bis 2500 mg/Liter.

Die Keimung wird bis zur Sprossbildung auf einem Medium durchgeführt, das eine organische Stickstoffquelle enthält. Zur Förderung des Längenwachstums der Wurzeln werden die Embryonen dann auf ein weiteres Medium überführt, das aber keine organische Stickstoffquelle enthält.

Die Dichte der Embryonen in diesem Medium wird so begrenzt, dass sie geringer ist als die Dichte, die dafür sorgt, dass die Entwicklung selbsthemmend ist. Geeignete Dichten sind beispielsweise 1 bis 100 Embryonen in einer 9 cm Petrischale, die etwa 10 bis 75 ml, bevorzugt 25 bis 50 ml, insbesonders etwa 35 ml Medium enthält.

Das Medium bzw. die Medien von Schritt (e) werden bei 20° bis 30°C gehalten. Die Temperatur beträgt vorzugsweise etwa 25°C (Raumtemperatur).

In Schritt (e) wird im allgemeinen etwas Licht benötigt. Geeignete Lichtintensitäten liegen zwischen 5 und 150 $\mu$E m$^{-2}$s$^{-1}$ (= 417,5 bis 12525 1x), vorzugsweise zwischen 10 und 75 $\mu$E m$^{-2}$s$^{-1}$ (= 835 bis 6262,5 1x).

Bis zur Keimung werden die Embryonen auf dem Medium bzw. den Medien von Schritt (e) gehalten; typischerweise 1 bis 20 Tage, üblicherweise 2 bis 4 Tage. Für den Fachmann ist ein gekeimter Embryo leicht erkennbar.

### Schritt f: Pflanzen

Nach der Keimung werden die Pflänzchen in Erde eingebracht und bis zur Reife weiterkultiviert. Die ausgepflanzten Pflänzchen werden anfangs mit Glas abgedeckt, um eine hohe Feuchtigkeit aufrechtzuerhalten. Nach etwa einer Woche unter Glas ist keine besondere Behandlung der Pflänzchen oder Pflanzen mehr nötig.

### Brauchbarkeit - Vermehrung

Reife Embryonen können für Massenvermehrung und Klonierung verwendet werden. Dazu bedarf es der Keimung der Embryonen und des Auspflanzens der Pflänzchen in Erde, in andere Wachstumssubstrate oder andere Wachstumsumgebungen. Reife Embryonen können auch von einem künstlichen Samenmantel umschlossen und als "somatische Samen" ausgesät werden. Massenvermehrung und Klonierung ist nutzbringend, wenn Hybrideltern oder ein Hybrid selbst in Massen produziert werden müssen.

Zellen, Proembryonen, Embryonen, Pflänzchen und Pflanzen können zu jeder Zeit während der oben beschriebenen Stadien analysiert werden, um zu bestimmen, ob irgendeine neue Eigenschaft als Folge genetischer Veränderungen vorhanden ist. Die Eigenschaft kann eine nützliche in vitro oder in planta Eigenschaft sein. Einige Beispiele von nützlichen Eigenschaften sind Phytotoxintoleranz, Trockentoleranz, Kältetoleranz, Krankheitstoleranz, etc.

Die aus den Schritten (a), (b) und (c) resultierenden Pflanzenzellen können auch in Gewebekulturverfahren eingesetzt werden, bei denen Pflanzen mit wünschenswerten Eigenschaften, wie beispielsweise Herbizidtoleranz, erzeugt werden sollen. Einige Beispiele solcher Verfahren sind beispielsweise in Chaleff und Ray in Science 223, 1148-1151 (1984) beschrieben.

Beispiele

Tabelle I - Medien

Alle Medien enthalten die bei Murashige und Skoog genannten anorganischen Salze und die bei Gamborg genannten B-5-Vitamine; sie sind auf einen pH von 5,7 eingestellt und haben die folgende Zusammensetzung (mg/Liter):

### Makronährstoffe

| | |
|---|---|
| $MgSO_4 \cdot 7H_2O$ | 370 |
| $KH_2PO_4$ | 170 |
| $KNO_3$ | 1900 |
| $NH_4NO_3$ | 1650 |
| $CaCl_2 \cdot 2H_2O$ | 440 |

### Mikronährstoffe

| | |
|---|---|
| $H_3BO_3$ | 6.2 |
| $MnSO_4 \cdot H_2O$ | 15.6 |
| $ZnSO_4 \cdot 7H_2O$ | 8.6 |
| $NaMoO_4 \cdot 2H_2O$ | 0.25 |
| $CuSO_4 \cdot 5H_2O$ | 0.025 |
| $CaCl_2 \cdot 6H_2O$ | 0.025 |
| $KI$ | 0.83 |
| $FeSO_4 \cdot 7H_2O$ | 27.8 |
| $Na_2EDTA$ | 37.3 |

### Vitamine

| | |
|---|---|
| Thiamin $\cdot$ HCl | 10 |
| Pyridoxin $\cdot$ HCl | 1 |
| Nicotinsäure | 1 |
| Myo-Inosit | 100 |

Zusätzlich enthalten die verschiedenen Medien die folgenden Komponenten:

```
Medium              Zusätzliche Komponenten

   1                20 g/Liter Saccharose, 0,6 % Agar Noble (Difco)

   2                30 g/Liter Glucose, 2 mg/l α-Naphthalinessigsäure,
                    1 mg/Liter Kinetin, 0,8 % Agar Noble

   3                30 g/Liter Saccharose, 2 mg/l α-Naphthalinessigsäure,
                    1 mg/Liter Kinetin, 0,8 % Agar Noble

   4                20 g/Liter Saccharose, 0,5 mg/l Picloram

   5                20 g/Liter Saccharose, 5 mg/l 2,4-Dichlorphenoxyessig-
                    säure

   6                20 g/Liter Saccharose, 15 mM Glutamin
```

Bei Medien, die bei 25°, 28° und 31°C eingesetzt werden, wird zusätzlich zur Temperatur, auf eine Photoperiode von 16 Stunden Licht/8 Stunden Dunkel, bei einer Lichtintensität von 20 $\mu$E m$^{-2}$s$^{-1}$ (= 1670 1x) Bezug genommen.

Beispiel 1: Samensterilisation und Auspflanzen

Samen von Baumwolle (*Gossypium hirsutum* var. Coker 310) werden entfasert, indem der Samen 2 Minuten in konzentrierte $H_2SO_4$ gelegt wird. Die Samen werden dann viermal mit sterilem, destilliertem Wasser gewaschen, in 95 % Ethanol getaucht, abgeflammt und auf Medium 1 bei 31°C gepflanzt.

Beispiel 2: Kallusinduktion

Sieben Tage nach dem Auspflanzen werden die Sämlinghypokotyle herausgeschnitten, längs aufgeschnitten, in 2 mm Abschnitte geschnitten und bei 31°C auf Medium 2 gelegt. Die Hypokotylabschnitte (2 mm) werden wöchentlich auf frisches Medium 2 übertragen, und diese Kulturen werden ebenfalls bei 31°C gehalten. Nach vier wöchentlichen Uebertragungen auf Medium 2 wird Kallusgewebe, das an den Hypokotylabschnitten proliferiert von dem ursprünglich verpflanzten Gewebe entfernt und auf Medium 3 bei 31°C gelegt. Der Kallus wird nach einem Monat auf frisches Medium 3 übertragen und für weitere ein bis zwei Monate unterhalten.

Beispiel 3: Initiierung der Suspensionskultur

Zur Initiierung von Suspensionskulturen werden 100 mg Kallusgewebe in 35 ml Medium 4 in einem 125 ml DeLong Gefäss gegeben. Die Suspensionen werden 6 Wochen mit 140 rpm (Umdrehungen pro Minute) und 28°C geschüttelt, während welcher Zeit sie anfangen, sich rasch zu vermehren.

Beispiel 4: Embryoentwicklung und Pflanzenregenerierung

Die sich im Medium 4 entwickelnden Embryonen vermehren sich noch schneller, wenn Medium 4 durch Medium 5 ersetzt wird. Die embryogene Suspension wird geteilt und alle 3 bis 7 Tage in frischem Medium 5 weitere Male kultiviert. Zur Entwicklung der sich in Medium 5 vermehrenden Embryonen werden die Embryonen mit Medium 6 gewaschen und anschliessend auch darein übertragen. Drei bis vier Wochen nach dem Transfer in Medium 6 werden die reifen Embryonen auf ein festes Medium bei 25°C gegeben. Das feste Medium besteht aus einem modifizierten MS-Medium, das MS Salze, das 40 mM $KNO_3$ an Stelle von $KNO_3$ und $NH_4NO_3$, sowie B-5-Vitamine, 2 % Saccharose, 15 mM Glutamin enthält und mit 0,2 % Gelrite verfestigt ist (pH 5,7). Die Embryonen werden in Petrischalen bei 25°C gegeben. Die Sprossentwicklung geschieht auf diesem Medium vereinzelt, und das Wurzellängenwachstum wird durch den Transfer der Embryonen auf das obige modifizierte MS-Medium ohne Glutamin verstärkt. Keimende Embryonen werden dann in Blähton in Tontöpfe gepflanzt und mit einem Becher bedeckt (25°C). Nachdem sich Pflänzchen im Blähton entwickelt haben, wird der Becher entfernt. Nach einer Woche bei 28°C werden die Pflänzchen in das Treibhaus in Erde gebracht, damit sie sich weiter zu ausgewachsenen Pflanzen entwickeln.

**Patentansprüche**

1. Verfahren, proembryonale Baumwollzellmassen in einer flüssigen Suspension herzustellen, das die folgenden Schritte enthält:

(a) Induktion der Baumwollkallusbildung bei etwa 20° bis 40°C, indem Baumwollpflanzengewebe auf ein Medium, das zur Kallusinduktion geeignet ist, aufgelegt wird, mit nachfolgenden weiteren Kultivierungsschritten zur Verhinderung von Bräunung;

(b) Suspension von bis zu 40 mg/ml des Kallus in einem geeigneten flüssigen Medium, das proembryonale Zellmassen induziert, welches eine relativ niedrige Konzentration von wenigstens einem Auxin enthält, bis sich klumpige Zusammenballungen proembryonaler Zellmassen bilden, die sich schnell vermehren; und dadurch gekennzeichnet, daß der zusätzliche Schritt durchgeführt wird:

(c) Uebertragung der sich rasch vermehrenden klumpigen Zusammenballungen der proembryonalen Zellmassen in ein flüssiges Nährmedium, in welchem kleinere, feiner dispergierte proembryonale Zellmassen gebildet werden können, wobei das besagte Nährmedium wenigstens ein Auxin enthält, in einer Konzentration, die höher ist als die die gewöhnlicherweise in Suspensionskulturen verwendet wird und die auf jeden. Fall bedeutend höher ist als die entsprechende Auseinkonzentration in Schritt b).

2. Verfahren, reife Baumwollembryonen in einer flüssigen Suspension herzustellen, dadurch gekennzeichnet, dass als Erweiterung des Verfahrens gemäss Anspruch 1 der zusätzliche Schritt durchgeführt wird: Entwicklung von torpedoförmigen oder keimblättrigen Embryonen aus den proembryonalen Zellmassen, indem die proembryonalen Zellmassen in ein zur Induktion von torpedoförmigen oder keimblättrigen Embryonen geeignetes Nährmedium gegeben werden.

3. Verfahren, Baumwollpflänzchen herzustellen, dadurch gekennzeichnet, dass als Erweiterung des Verfahrens gemäss Anspruch 2 der zusätzliche Schritt durchgeführt wird: Auskeimen der Embryonen auf einem geeigneten Embryoauskeimungsnährmedium.

4. Verfahren, Baumwollpflanzen herzustellen, dadurch gekennzeichnet, dass als Erweiterung des Verfahrens gemäss Anspruch 3 der zusätzliche Schritt durchgeführt wird: Aufzucht von ausgewachsenen Pflanzen aus den gekeimten Embryonen.

5. Verfahren gemäss Anspruch 1, 2, 3 oder 4, dadurch gekennzeichnet, dass die Baumwolle *Gossypium hirsutum* ist.

6. Verfahren gemäss Anspruch 1, 2, 3 oder 4, dadurch gekennzeichnet, dass das Baumwollpflanzengewebe in Schritt (a) Keimblatt- oder Hypokotylgewebe ist.

7. Verfahren gemäss Anspruch 1, 2, 3 oder 4, dadurch gekennzeichnet, dass das Auxin in Schritt (b) Picloram in einer Konzentration von 0,1 bis 5 mg/Liter ist.

8. Verfahren gemäss Anspruch 1, 2, 3 oder 4, dadurch gekennzeichnet, dass das Auxin in Schritt (b) 2,4-Dichlorphenoxyessigsäure in einer Konzentration von 0,01 bis 0,5 mg/Liter ist.

9. Verfahren gemäss Anspruch 1, 2, 3 oder 4, dadurch gekennzeichnet, dass Schritt (b) 3 bis 8 Wochen lang durchgeführt wird.

10. Verfahren gemäss Anspruch 1, 2, 3 oder 4, dadurch gekennzeichnet, dass bis zu 15 mg/ml Kallus im Medium von Schritt (b) suspendiert wird.

11. Verfahren gemäss Anspruch 1, 2, 3 oder 4, dadurch gekennzeichnet, dass das Auxin in Schritt (c) 2,4-Dichlorphenoxyessigsäure in einer Konzentration von 0,5 bis 100 mg/Liter ist.

12. Verfahren gemäss Anspruch 2, 3 oder 4, dadurch gekennzeichnet, dass das Medium von Schritt (d) anorganische Salze, Vitamine, eine Kohlenstoffquelle und eine organische Verbindung enthält, die reduzierten Stickstoff enthält.

13. Verfahren gemäss Anspruch 12, dadurch gekennzeichnet, dass die Verbindung, die reduzierten Stickstoff enthält, Glutamin ist, in einer Konzentration von 2 bis 250 mM.

**Claims**

1. A method for producing pro-embryonic cotton cell masses in a liquid suspension, which consists of the following steps:

(a) inducing cotton callus formation at about 20 to 40°C by placing cotton plant tissue on a medium suitable for callus induction, with subsequent further culturing steps to prevent browning;

(b) suspending up to 40 mg of the callus/ml in a suitable liquid medium which induces pro-embryonic cell masses and comprises a relatively low concentration of at least one auxin, until clumpy aggregates of pro-embryonic cell masses form and begin rapidly to proliferate; and which comprises carrying out the additional step:

EP 0 317 512 B1

(c) transferring the rapidly proliferating clumpy aggregates of pro-embryonic cell masses to a liquid nutrient medium in which smaller, more finely dispersed pro-embryonic cell masses are able to form, the said nutrient medium comprising at least one auxin at a concentration higher than that conventionally employed in suspension cultures and in any case significantly higher than the corresponding auxin concentration in step (b).

2. A method for producing mature cotton embryos in a liquid suspension, which comprises carrying out, as an extension of the method according to claim 1, the additional step: (d) developing torpedo or cotyledonous embryos from the pro-embryonic cell masses by placing the pro-embryonic cell masses in a nutrient medium suitable for inducing torpedo or cotyledonous embryos.

3. A method for producing cotton plantlets, which comprises carrying out, as an extension of the method according to claim 2, the additional step: germinating the embryos on a suitable embryo germination nutrient medium.

4. A method for producing cotton plants, which comprises carrying out, as an extension of the method according to claim 3, the additional step: cultivation of fully grown plants from the germinated embryos.

5. A method according to claim 1, 2, 3 or 4, wherein the cotton is *Gossypium hirsutum.*

6. A method according to claim 1, 2, 3, or 4, wherein the cotton plant tissue in step (a) is cotyledon or hypocotyl tissue.

7. A method according to claim 1, 2, 3 or 4, wherein the auxin in step (b) is picloram at a concentration of 0.1-5 mg/litre.

8. A method according to claim 1, 2, 3 or 4, wherein the auxin in step (b) is 2,4-dichlorophenoxyacetic acid at a concentration of 0.01-0.5 mg/litre.

9. A method according to claim 1, 2, 3 or 4, wherein step (b) is carried out for 3 to 8 weeks.

10. A method according to claim 1, 2, 3 or 4, wherein up to 15 mg of callus/ml is suspended in the medium of step (b).

11. A method according to claim 1, 2, 3 or 4, wherein the auxin in step (c) is 2,4-dichlorophenoxyacetic acid at a concentration of 0.5 to 100 mg/litre.

12. A method according to claim 2, 3, or 4, wherein the medium of step (d) comprises inorganic salts, vitamins, a carbon source and an organic compound containing reduced nitrogen.

13. A method according to claim 12, wherein the compound containing reduced nitrogen is glutamine at a concentration of 2 to 250 mM.


**Revendications**

1. Procédé pour la production de masses cellulaires de coton proembryonnaires dans une suspension fluide, comprenant les étapes suivantes:
(a) induction de la formation de cals de coton à environ 20° à 40°C, tandis qu'on dispose du tissu de cotonnier sur un milieu approprié pour l'induction de cal, avec d' autres étapes de culture subséquentes pour éviter le brunissement;
(b) mise en suspension de jusqu'à 40 mg/ml du cal dans un milieu liquide approprié qui induit des masses cellulaires proembryonnaires, contenant une concentration relativement faible d'au moins une auxine,,jusqu'à ce qu'il se forme des précipités grumeleux de masses cellulaires proembryonnaires, qui se multiplient rapidement; et caractérisé en ce qu'on effectue l'étape supplémentaire de:
(c) transfert des précipités grumeleux des masses cellulaires proembryonnaires se multipliant rapidement dans un milieu nutritif liquide, dans lequel on peut former des masses cellulaires proembryonnaires plus petites, finement dispersées, tandis que le milieu nutritif susdit contient au moins une auxine, en une concentration qui est supérieure à celle qui est utilisée d'ordinaire dans les milieux de culture en suspension et qui est en tous cas nettement supérieure à la concentration correspondante d'auxine dans l'étape b).

2. Procédé pour la production d'embryons mûrs de coton dans une suspension fluide, caractérisé en ce que comme extension du procédé selon la revendication 1 on effectue l'étape supplémentaire de: développement d'embryons en forme de torpille ou de cotylédons à partir des masses cellulaires proembryonnaires, tandis qu'on place les masses cellulaires pro-embryonnaires dans un milieu nutritif approprié pour l'induction d'embryons en forme de torpille ou de cotylédons.

3. Procédé pour la production de plantules de coton, caractérisé en ce que comme extension du procédé selon la revendication 2 on effectue l'étape supplémentaire de: germination des embryons sur un milieu de germination d'embryons approprié.

4. Procédé pour la production de plants de coton,, caractérisé en ce que comme extension du procédé selon la revendication 3 on effectue l'étape supplémentaire de: développement de plantes germées à partir

11

d'embryons germés.

5. Procédé selon l'une quelconque des revendications 1, 2, 3 ou 4, caractérisé en ce que le coton est du *Gossypium hirsutum*.

6. Procédé selon l'une quelconque des revendications 1, 2, 3 ou 4, caractérisé en ce le tissu végétal de coton dans l'étape (a) est du tissu de cotylédon ou d'hypocotyle.

7. Procédé selon l'une quelconque des revendications 1, 2, 3 ou 4, caractérisé en ce que l'auxine dans l'étape (b) est du Picloram en une concentration de 0,1 à 5 mg/litre.

8. Procédé selon l'une quelconque des revendications 1, 2, 3 ou 4, caractérisé en ce que l'auxine dans l'étape (b) est de l'acide 2,4-dichlorophénoxyacétique en une concentration de 0,01 à 0,5 mg/litre.

9. Procédé selon l'une quelconque des revendications 1, 2, 3 ou 4, caractérisé en ce qu'on exécute l'étape (b) pendant 3 à 8 semaines.

10. Procédé selon l'une quelconque des revendications 1, 2, 3 ou 4, caractérisé en ce qu'on met en suspension jusqu'à 15 mg/ml de cals dans le milieu de l'étape (b).

11. Procédé selon l'une quelconque des revendications 1, 2, 3 ou 4, caractérisé en ce que l'auxine dans l'étape (c) est de l'acide 2,4-dichlorophénoxyacétique en une concentration de 0,5 à 100 mg/litre.

12. Procédé selon l'une quelconque des revendications 2, 3 ou 4, caractérisé en ce que le milieu de l'étape (d) contient des sels minéraux, des vitamines, une source de carbone et un composé organique contenant de l'azote réduit.

13. Procédé selon la revendication 12, caractérisé en ce que le composé contenant de l'azote réduit est de la glutamine en une concentration de 2 à 250 mM.

Fig. 1/8

Fig. 2/8

Fig. 3/8

Fig. 4/8

Fig. 5/8

Fig. 6/8

Fig. 7/8

Fig. 8/8